# EUROPEAN PATENT SPECIFICATION

(11) **EP 1 011 542 B1**
(45) Date of publication and mention of the grant of the patent: **27.09.2006**
(21) Application number: 98931344.0
(22) Date of filing: 17.06.1998
(51) Int. Cl.: A61F 2/38

(54) **Joint endoprosthesis**
Gelenkendprothese
Endoprothèse d' articulation

(30) Priority: 20.06.1997 US 50291 P
(43) Date of publication of application: 28.06.2000
(73) Proprietor: BIOMEDICAL ENGINEERING TRUST I, South Orange, New Jersey 07079 (US)
(72) Inventor: Pappas, Michael J., Caldwell, NJ 07006 (US); Buechel, Frederick F., South Orange, NJ 07079 (US)
(74) Representative: Müller-Boré & Partner Patentanwälte
(86) International application number: PCT/US1998/012637
(87) International publication number: WO 1998/058603

(56) References cited:
- FR-A- 2 676 916
- FR-A- 2 700 263
- US-A- 4 207 627
- US-A- 4 340 978
- US-A- 4 711 639
- US-A- 4 714 474
- US-A- 5 080 675
- US-A- 5 282 868
- US-A- 5 470 354

## Description

### BACKGROUND OF THE INVENTION

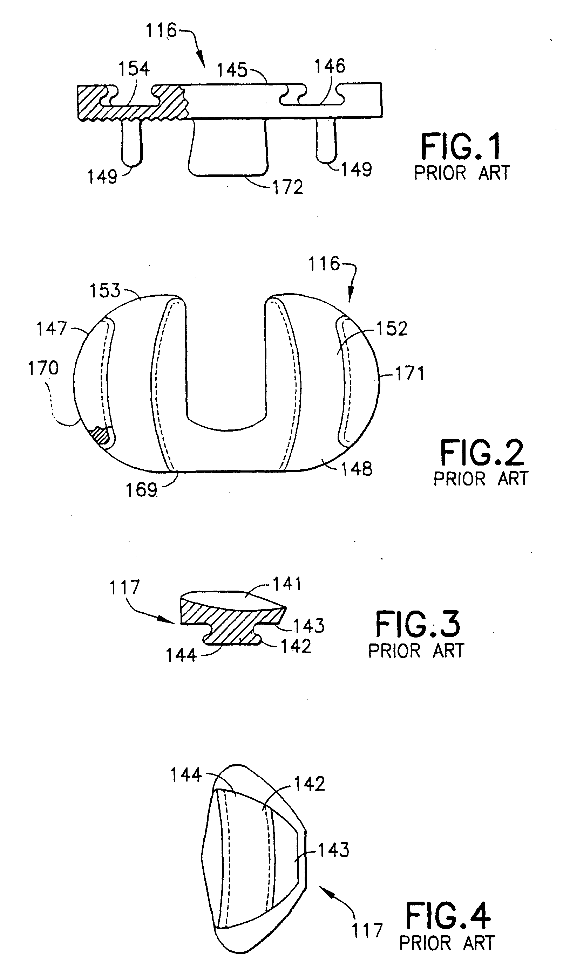

A well-known bearing platform and meniscal bearing combination is disclosed in U.S. Patent Nos. 4,309,778, and 4,340,978, to the same inventors of this application. U.S. Patent Nos. 4,309,778 and 4,340,978 disclose knee prostheses as depicted in FIGS. 1-4. Briefly, referring to FIGS. 1-4 herein, a tibial platform 116 is formed to define two curved tracks 148, 153. Each of the tracks 148, 153 is formed by raised portions 169 and 171, and 169 and 170, respectively, and cross-sectionally defines a dovetail configuration. Projections 149, 172 extend from the inferior surface of the platform 116 and are provided to help distribute joint loads, supply additional load transfer to the cancellous bone, and provide resistance against possible tensile loading. Additionally, two tibial bearing components 117 are also provided to be disposed in the tracks 148, 153. As shown in FIGS. 3-4, each of the bearing elements 117 is formed with a primary load bearing surface 141 on its superior side and a second bearing surface 142 on its inferior side. Each of the second bearing surfaces 142 includes a flat surface 143 and a projecting dovetail surface 144. The dovetail surface 144 is cross-sectionally formed to substantially the same dimensions as the dovetail configuration defined by each of the tracks 148, 153. As can be seen in FIG. 4, the dovetail surface 144 extends longitudinally over a substantial portion of the length of the bearing component 117 and is curved with the same radius of curvature as each of the respective tracks 148, 153. It is to be understood, that the tracks 148, 153 curve symmetrically about the platform 116, and, each of the bearings is formed to curve in a similar fashion. With the dovetail surfaces 142 of the bearing components 117 being interengaged with the dovetail configurations of the tracks 148, 153, anterior-posterior shift of the bearing elements 117 relative to the platform 116 is attainable.

As a further example, FR-2 700 263 relates to a prosthetic joint comprising a tibial platform with a superior and an inferior bearing surface, two bearing elements having a convex surface and a femoral component, which is adapted to slide on the bearing elements.

### SUMMARY OF THE INVENTION

To meet the above-identified need, a combination of a new and improved meniscal bearing platform and bearing components is provided for use in a prosthetic joint. The combination is used in conjunction with a femoral component. The subject invention is a prosthetic joint according to claim 1.

The prior art bearing platform, as shown in FIGS. 1-2, is formed for mounting onto a resected tibia. The platform of the subject application does not include the lateral track walls that had been included in the prior art bearing platform and that are designated by reference numerals 170, 171 in FIG. 2. Rather the platform of the subject invention includes a central raised portion having curved longitudinal edges which are each formed with a dovetail type configuration.

Two bearing components are provided, each formed with a bearing surface on its superior side, and a bearing surface on its inferior side. For each of the bearing components, only a partial dovetail configuration is formed along a single edge thereof. Specifically, each of the bearing elements is formed with an inner edge having a dovetail configuration formed therein which mates with one of the edges of the raised portion formed on the platform. The mating dovetail configurations provide dislocation resistance for the platform and bearing elements combination. The outer edges, however, of the bearing elements are formed smoothly, with no such dovetail configuration. Consequently, the inferior bearing surface of each of the bearing elements, which is in contact with the platform, is larger in area as compared to the prior art design. Also the bearing elements of the subject invention are each formed thicker in a superior to inferior direction in the region of highest loading as compared to the prior art design. This greater thickness is attributable to the absence of a dovetail at the lateral and medial extremes of the prosthesis. Thus, this design is more robust than the prior art.

The movement of the bearing components relative to the central raised portion of the tibial platform is constrained by the femoral component. The femoral component, prevents radially outward movement of the bearing components whereas, the central raised portion prevents radially inward movement of the bearing components.

These and other features of the invention will be better understood through a study of the following detailed description and accompanying drawings.

### BRIEF DESCRIPTION OF THE DRAWINGS

FIG. 1 is a partial cross-sectional view of a prior art tibial platform. FIG. 1 is taken from FIG. 15 of U.S. Patent No. 4,309,778.
FIG. 2 is a plan view of the prior art tibial platform. FIG. 2 is taken exactly from FIG. 16 of U.S. Patent No. 4,309,778.
FIG. 3 is a cross-sectional view of a prior art bearing element. FIG. 3 is taken from FIG. 19 of U.S. Patent No. 4,309,778.
FIG. 4 is a bottom plan view of the prior art bearing element. FIG. 4 is taken from FIG. 21 of U.S. Patent No. 4,309,778.
FIG. 5 is a top plan view of the new and improved tibial platform of the subject application.
FIG. 6 is a bottom plan view of the new and improved tibial platform of the subject application.
FIG. 7(A) and 7(B) are top and bottom views of the new and improved bearings of the subject application.
FIG. 8 is a plan view of the bearings disposed on the tibial platform of the subject application.
FIG. 9 is a side view of a prosthesis including the tibial platform and bearing elements of the subject application.
FIG. 10 is a front plan view of a prosthesis including the tibial platform and bearing elements of the subject application.

### DETAILED DESCRIPTION OF THE PREFERRED EMBODIMENTS

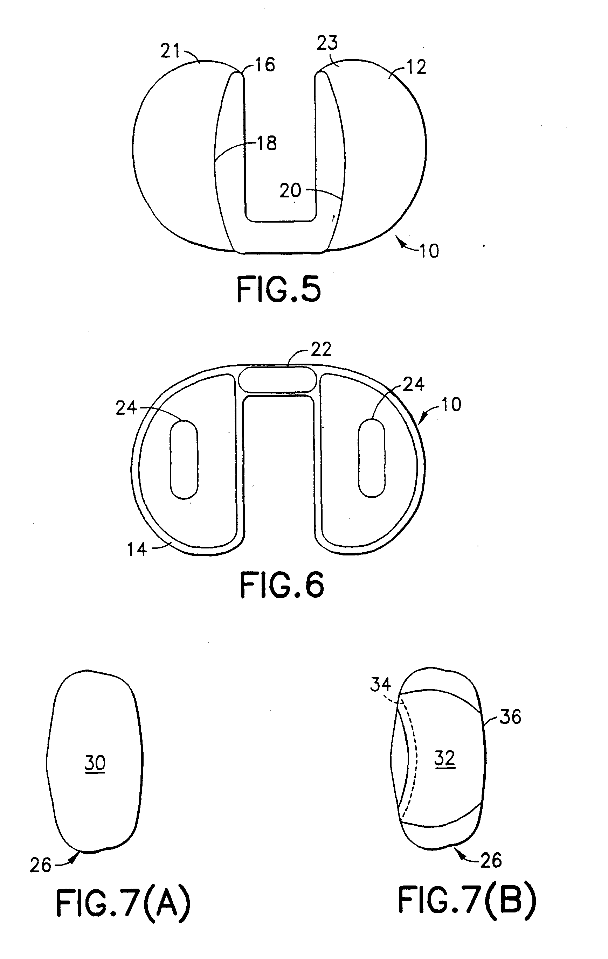

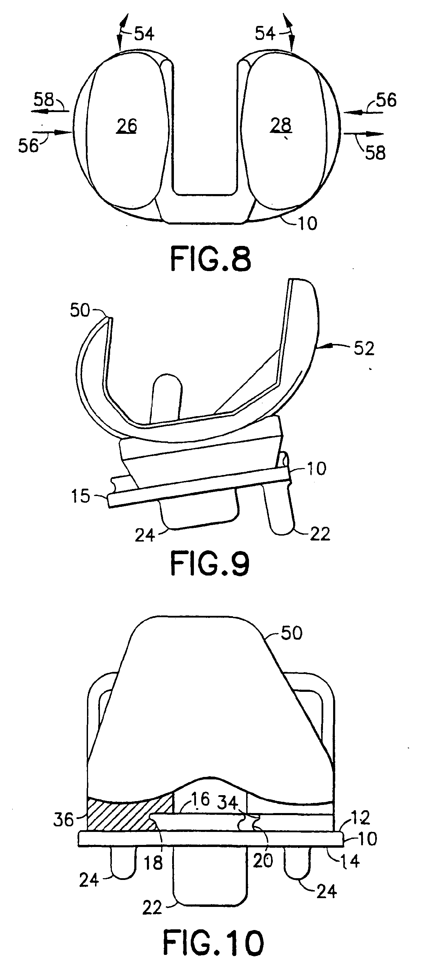

Referring generally to FIGS. 5, 6 and 9, a tibial platform 10 is provided having a superior surface 12, an inferior surface 14 and an edge surface 15. As can be seen from FIG. 10, a raised portion 16 extends centrally from the superior side 12 of the platform 10 in an anterior-posterior direction. The side edges 18, 20 of the raised portion 16, as shown in FIG. 10, each are formed to define an arcuate dovetail-type surface. Referring to FIG. 5, the side edges 18, 20 are curved in opposite directions along the longitudinal length of the platform 10. The tibial platform 10, the raised portion 16, and the side edges 18, 20 are formed in accordance with the teachings of U.S. Patent Nos. 4,309,778 and 4,340,978. Surface portions 21, 23, respectively, extend laterally from the raised portion 16 to the portions of the edge surface 15 located at lateral-most portions of the tibial platform 10. Surface portions 21, 23 are planar. As can be seen from FIGS. 9-10, projections 22,24 extend from the inferior surface 14 of the platform 10. The projections 22, 24 are also formed in accordance with U.S. Patent Nos. 4,309,778 and 4,340,978.

Two bearing elements 26, 28 are also provided, which are preferably formed from a thermoplastic material. Each of the bearing elements 26, 28 has a superior bearing surface 30 and an inferior bearing surface 32. Although reference is only to the bearing element 26 in FIGS. 7(A) and 7(B), it is to be understood the bearing element 28 is formed identically. As best can be seen in FIG. 10, the bearing elements 26, 28 each are formed with an inner edge 34 having substantially the same dovetail-type configuration as the side edges 18, 20 of the raised portion 16 of the platform 10, but are oriented as a mirror image, to allow face-to-face engagement of the side edges 18 and 34, and inner edges 20 and 34, respectively. As shown in FIGS. 7(A) and 7(B), the inner edges 34 of the bearing elements 26, 28 are curved in a similar fashion to the side edges 18, 20 of the raised portion 16 of the platform 10. Consequently, as shown in FIGS. 8-10, the bearing elements 26, 28 are disposed on the platform 10 with the respective inner edges 34 in mating contact with the side edges 18, 20, respectively, of the raised portion 16, with the inferior bearing surfaces 32 of the bearing elements 26, 28 being in face-to-face engagement with the superior surface 12 of the platform 10. Each of the bearing elements 26, 28 is also formed with an outer edge 36 which is not formed with a dovetail configuration and is preferably flat. As shown in FIG. 10, since the outer edge 36 is not formed with a dovetail configuration, the thickness of solid material forming the bearing elements 26, 28, as measured between the respective superior bearing surfaces 30 and the inferior bearing surfaces 32, is greater in proximity to the outer edge 36 than to the inner edge 34.

The superior bearing surfaces 30 of the bearing elements 26, 28 are formed to engage in articulation with at least portions of a bearing surface 52 of a femoral component 50. The femoral component 50 constrains movement of the bearing elements 26, 28 about the raised portion 16. The bearing surface 52 of the femoral component 50 and the respective superior bearing surfaces 30 of the bearing elements 26, 28 are formed as in U.S. Patent Nos. 4,309,778 and 4,340,978 and as such the interengagement therebetween provides lateral constraint for the bearing elements 26, 28. With reference to FIG. 8, the bearing elements 26, 28 may move along a path defined adjacent the raised portion 16, as designated by the arrow 54, but not in radially inward directions (as represented by arrow 56) due to constraint by the raised portion 16, or in radially outward directions (as represented by arrow 58) due to constraint by the femoral component 50.

## Claims

1. A prosthetic joint comprising:
a tibial platform (10) having a first superior bearing surface (12), a first inferior bearing surface (14), and an edge surface (15) extending therebetween, said first inferior bearing surface (14) being formed for attachment to a bone, said first superior bearing surface (12) being formed with an elongated raised portion (16) extending therefrom, said raised portion (16) extending in an anterior posterior direction and formed to define curved side edges (18, 20), and said first superior bearing surface (12) being formed with planar surface portions (21, 23) extending laterally from said raised portion (16) to portions of said edge surface (15) located at lateral-most portions of said tibial platform (10);
two bearing elements (26, 28), each having a second superior bearing surface (30) and a second inferior bearing surface (32), said second inferior bearing surface (32) being planar and formed to slidably engage said first superior bearing surface (12) of said tibial platform (10) for sliding movement relative thereto, and wherein said bearing elements (26, 28) are each formed with an inner edge (34) and an outer edge (36), each said inner edge (34) being disposed to face said raised portion and being curved and defining the same radius of curvature as said side edges (18, 20), and wherein each said bearing element (26, 28) is formed with a greater thickness of solid material adjacent to said outer edge than said inner edge, said thickness being measured in a direction extending between said second superior bearing surface (30) and said inferior bearing surface (32); and
a femoral component (50) having a third superior bearing surface (52) formed for sliding articulation with said second superior bearing surfaces (30) of said bearing elements (26, 28), said femoral component (50) being formed to constrain sliding movement of said bearing elements (26, 28) to a predetermined path relative to said raised portion.

2. A prosthetic joint according to claim 1, wherein said inner edges (34) of said bearing elements (26, 28) are in dovetail engagement with said side edges (18, 20) of said raised portion (16).

3. A prosthetic joint according to claim 2, wherein said predetermined path of sliding movement of said bearing elements (26, 28) is curvilinear, wherein said raised portion (16) constrains movement of said bearing elements (26, 28) in radially inward directions relative to said predetermined path, and wherein said femoral component (50) constrains movement of said bearing elements (26, 28) in radially outward directions relative to said predetermined path.

## Patentansprüche

1. Gelenkprothese, umfassend:
eine Tibialplattform (10), die eine erste obere Lageroberfläche (12), eine erste untere Lageroberfläche (14) und eine Rand- bzw. Kantenoberfläche (15) aufweist, die sich dazwischen erstreckt, wobei die erste untere Lageroberfläche (14) zur Festlegung an einem Knochen ausgebildet ist, wobei die erste obere Lageroberfläche (12) mit einem verlängerten, angehobenen Abschnitt (16) ausgebildet ist, der sich davon erstreckt, wobei sich der angehobene Abschnitt (16) in einer Vorwärts/Rückwärtsrichtung erstreckt und ausgebildet ist, um gekrümmte Seitenkanten bzw. -ränder (18, 20) zu definieren, und wobei die erste obere Lageroberfläche (12) mit planaren bzw. ebenen Oberflächenabschnitten (21, 23) ausgebildet ist, die sich seitlich von dem angehobenen Abschnitt (16) zu Abschnitten der Kantenoberfläche (15) erstrecken, die an den am weitesten seitlichen Abschnitten der Tibialplattform (10) angeordnet sind;
zwei Lagerelemente (26, 28), wobei jedes eine zweite obere Lageroberfläche (30) und eine zweite untere Lageroberfläche (32) aufweist, wobei die zweite untere Lageroberfläche (32) planar bzw. eben ist und ausgebildet ist, um gleitbar die erste obere Lageroberfläche (12) der Tibialplattform (10) für eine gleitende Bewegung relativ dazu zu ergreifen, und wobei die Lagerelemente (26, 28) jeweils mit einer Innenkante (34) und einer Außenkante (36) ausgebildet sind, wobei jede Innenkante (34) angeordnet ist, um zu dem angehobenen Abschnitt gerichtet zu sein, und gekrümmt ist und denselben Krümmungsradius wie die Seitenkanten (18, 20) definiert, und wobei jedes Lagerelement (26, 28) mit einer größeren Dicke an festem Material benachbart zu der Außenkante als die Innenkante ausgebildet ist, wobei die Dicke in einer Richtung gemessen ist, die sich zwischen der zweiten oberen Lageroberfläche (30) und der unteren Lageroberfläche (32) erstreckt; und
eine Femoralkomponente (50), die eine dritte obere Lageroberfläche (52) aufweist, die für eine gleitende Gelenkverbindung mit der zweiten oberen Lageroberfläche (30) der Lagerelemente (26, 28) ausgebildet ist, wobei die Femoralkomponente (50) ausgebildet ist, um eine Gleitbewegung der Lagerelemente (26, 28) auf einen vorbestimmten Pfad relativ zu dem angehobenen Abschnitt zu beschränken.

2. Gelenkprothese nach Anspruch 1, wobei die Innenkanten (34) der Lagerelemente (26, 28) in einem Schwalbenschwanzeingriff mit den Seitenkanten (18, 20) des angehobenen Abschnitts (16) sind.

3. Gelenkprothese nach Anspruch 2, wobei der vorbestimmte Pfad der Gleitbewegung der Lagerelemente (26, 28) gekrümmt ist, wobei der angehobene Abschnitt (16) eine Bewegung der Lagerelemente (26, 28) in radial nach innen verlaufenden Richtungen relativ zu dem vorbestimmten Pfad beschränkt, und wobei die Femoralkomponente (50) eine Bewegung der Lagerelemente (26, 28) in radial nach außen gerichteten Richtungen relativ zu dem vorbestimmten Pfad beschränkt.

## Revendications

1. Articulation prothétique comprenant :
une plate-forme tibiale (10) ayant une première surface de portée supérieure (12), une première surface de portée inférieure (14) et une surface de bord (15) s'étendant entre les précédentes, la dite première surface de portée inférieure (14) étant formée pour fixation à un os, la dite première surface de portée supérieure (12) étant formée avec une partie surélevée allongée (16) qui s'étend à partir de cette surface (12), la dite partie surélevée (16) s'étendant dans une direction antérieure - postérieure et étant formée de manière à définir des bords latéraux courbes (18, 20), et la dite première surface de portée supérieure (12) étant formée avec des parties de surface planes (21, 23) qui s'étendent latéralement à partir de la dite partie surélevée (16) jusqu'à des parties de la dite surface de bord (15) situées aux endroits les plus latéraux de la dite plate-forme tibiale (10) ;
deux éléments de portée (26, 28), présentant chacun une deuxième surface de portée supérieure (30) et une deuxième surface de portée inférieure (32), la dite deuxième surface de portée inférieure (32) étant plane et formée de manière à venir en contact glissant avec la dite première surface de portée supérieure (12) de la dite plate-forme tibiale (10) pour un mouvement de glissement par rapport à cette dernière, et les dits éléments de portée (26, 28) sont formés chacun avec un bord intérieur (34) et un bord extérieur (36), chaque dit bord intérieur (34) étant disposé en face de la dite partie surélevée et étant incurvé et définissant le même rayon de courbure que les dits bords latéraux (18, 20), et chaque dit élément de portée (26, 28) est formé avec une épaisseur de matière solide plus grande près du dit bord extérieur qu'à l'endroit du dit bord intérieur, la dite épaisseur étant mesurée dans une direction s'étendant entre la dite deuxième surface de portée supérieure (30) et la dite surface de portée inférieure (32) ; et
un composant fémoral (50) ayant une troisième surface de portée supérieure (52) formée pour une articulation glissante avec les dites deuxièmes surfaces de portée supérieures (30) des dits éléments de portée (26, 28), le dit composant fémoral (50) étant formé de manière à limiter le mouvement glissant des dits éléments de portée (26, 28) à un chemin prédéterminé par rapport à la dite partie surélevée.

2. Articulation prothétique selon la revendication 1, dans laquelle les dits bords intérieurs (34) des dits éléments de portée (26, 28) sont en contact en queue d'aronde avec les dits bords latéraux (18, 20) de la dite partie surélevée (16).

3. Articulation prothétique selon la revendication 2, dans laquelle le dit chemin prédéterminé de mouvement glissant des dits éléments de portée (26, 28) est curviligne, dans laquelle la dite partie surélevée (16) empêche un mouvement des dits éléments de portée (26, 28) dans des directions radialement vers l'intérieur par rapport au dit chemin prédéterminé, et dans laquelle le dit composant fémoral (50) empêche un mouvement des dits éléments de portée (26, 28) dans des directions radialement vers l'extérieur par rapport au dit chemin prédéterminé.
